# EUROPÄISCHE PATENTSCHRIFT

(11) **EP 0 693 466 B1**
(45) Veröffentlichungstag und Bekanntmachung des Hinweises auf die Patenterteilung: **17.10.2001**
(21) Anmeldenummer: 95110781.2
(22) Anmeldetag: 11.07.1995
(51) Int. Cl.: C07B 39/00, C07C 17/20, C07C 205/12, C07C 233/36, C07C 233/18, C07D 207/26, C07D 233/36

(54) **Verfahren zur Herstellung von aromatischen fluorierten Verbindungen und neue Diamide**
Process for the preparation of aromatic fluorinated compounds and diamides
Procédé pour la préparation de composés aromatiques fluorés et diamides

(30) Priorität: 22.07.1994 DE 4426133
(43) Veröffentlichungstag der Anmeldung: 24.01.1996
(73) Patentinhaber: BAYER AG, 51368 Leverkusen (DE)
(72) Erfinder: Marhold, Albrecht, Dr., D-51373 Leverkusen (DE); Löhr, Marianne, B26 6BT, Selly Oak, Birmingham (GB); Wamhoff, Heinrich, Prof.Dr., D-53757 St. Augustin (DE)

(56) Entgegenhaltungen:
- EP-A- 0 371 563
- DE-A- 2 515 146
- FR-A- 2 391 990
- GB-A- 1 360 327
- DATABASE CROSSFIRE Beilstein Informationssysteme Gmbh Frankfurt DE, & JUSTUS LIEBIGS ANNALEN DER CHEMIE, Bd. 596, 1955 WEINHEIM DE,
- DATABASE CROSSFIRE Beilstein Informationssysteme Gmbh Frankfurt DE, & RECUEIL DES TRAVAUX CHIMIQUES DES PAYS-BAS, Bd. 57, 1938 AMSTERDAM NL,
- DATABASE CROSSFIRE Beilstein Informationssysteme Gmbh Frankfurt DE, & J.MED.PHARM.CHEM., Bd. 5, 1962 Seiten 823-835,
- JOURNAL OF THE AMERICAN CHEMICAL SOCIETY, Bd. 79, 1957 DC US, Seiten 734-741, R. GHIRARDELLI ET AL. 'Stereochemistry of the Opening of the Imine Ring with Ethylamine'

## Beschreibung

Die Halogenaustauschreaktion (= Halex-Reaktion) zur Fluorierung von aromatischen Halogenverbindungen wird im allgemeinen in polaren aprotischen Lösungsmitteln wie Dimethylformamid, Dimethylacetamid, N-Methylpyrrolidon, Tetramethylensulfon oder Dimethylsulfoxid durchgeführt. Nachteilig sind häufig die schlechten Löslichkeiten der Fluorierungsmittel (z.B. Kaliumfluorid) in den bekannten Lösungsmitteln und die dadurch bedingten hohen Reaktionstemperaturen. Beim Einsatz von Halogen-Nitroverbindungen besteht bei hohen Reaktionstemperaturen ein erhebliches Risiko von spontanen Zersetzungen. Außerdem erfordern derartige Umsetzungen lange Reaktionszeiten.

Deshalb ist es wünschenswert, Lösungsmittel für Halex-Reaktionen zur Verfügung zu haben, die größere Anteile von Fluorierungsmitteln zu lösen vermögen. In solchen Lösungsmitteln könnte die Halex-Reaktion bei niedrigerer Temperatur und in kürzerer Zeit ablaufen, technisch einfacher durchgeführt werden und bestünde praktisch kein Risiko mehr von spontanen Zersetzungen.

Aus Acta Chem. Scand. B31, 399 bis 406 (1977) sind N,N'-Dimethyl-N,N'-diacetylethylendiamin und N,N'-Dirnethyl-N,N'-diacetylpropan-1,3-diamin und physikalischchemische Messungen daran bekannt.

Aus Reppe et al., Justus Liebigs Ann. Chem. 596 (1955), 203 und 212 sind N,N'-3-Oxapentandiyl-bis-pyrrolidin-2-on und N,N'-Ethandiyl-bis-pyrrolidin-2-on bekannt.

Aus DE-A-25 15 146 sind N,N'-Dimethyl-N,N'-ethandiyl-bis-propionamid, N,N'-Dimethyl-N,N'-ethandiyl-bis-isobutyramid, N,N'-Dimethyl-N,N'-ethandiyl-bis-valeramid, N,N'-Dimethyl-N,N'-ethandiyl-bis-hexancarboxamid und N,N'-Dimethyl-N,N'-ethandiyl-bis-1-ethyl-butyramid bekannt.

Aus Rameau et al., Recl. Trav. Chim. Pays-Bas 57 (1938), 208 ist N,N'-Diisobutyl-N,N'-ethandiyl-bis-acetamid bekannt.

Aus Shepherd et al., J. Med. Pharm. Chem. 5 (1962), 823-835 ist N,N'-Diisopropyl-N,N'-ethandiyl-bis-acetamid bekannt.

Aus Ghirardelli et al., JACS 79 (1957), 734-741 ist N,N'-Diethyl-N,N'-butan-(2,3)-diylbis-acetamid bekannt.

GB-A-13 60 327 beschreibt ein Verfahren zur Herstellung aromatischer fluorierter Verbindungen durch Halogenaustauschreaktion, in welchem als Lösungsmittel Dimethylacetamid eingesetzt wird.

EP-A-0 371 563 offenbart ein Verfahren zur Herstellung von Chlorfluorbenzolen und Difluorbenzolen durch Halogenaustauschreaktion in polaren, aprotischen Lösungsmitteln.

FR-A-23 91 990 beschreibt ein Verfahren zur Herstellung von m-Nitrofluorbenzolen und m-Cyanofluorbenzolen aus den entsprechenden Chlorverbindungen durch Halogenaustauschreaktion, wobei als Lösungsmittel Tetramethylsulfon oder Dimethylformamid eingesetzt werden.

Es wurde nun ein Verfahren zur Herstellung von aromatischen, am Kern fluorierten Verbindungen durch Umsetzung von entsprechenden Chlor- oder Bromverbindungen mit Kaliumfluorid in einem Lösungsmittel gefunden, das dadurch gekennzeichnet ist, daß man als Lösungsmittel ein Diamid einsetzt.

Beispielsweise kann man in das erfindungsgemäße Verfahren ein Diamid der Formel (I) einsetzen 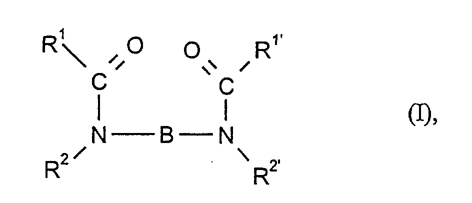 in der
- B: für eine Brücke der Formel (II) steht 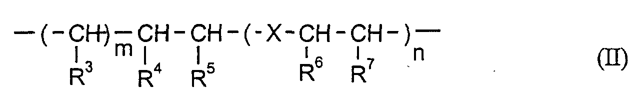 mit R³, R⁴, R⁵, R⁶ und R⁷ unabhängig voneinander jeweils gleich Wasserstoff oder C₁-C₆-Alkyl,
X= Sauerstoff, Schwefel oder N-C₁-C₆-Alkyl,
m= Null oder 1 und
n= Null, 1 oder 2,
- R¹ und R²: unabhängig voneinander für C₁-C₆-Alkyl oder gemeinsam für eine Brücke der Formeln
-(-CH₂-)₃-, -(-CH₂-)₄-, -Y-(-CH₂-)₂-, -Y-(-CH₂-)₃-, -(-CH₂-)-Y-(-CH₂-)-, -(-CH₂-)-Y-(-CH₂-)₂- oder -(-CH₂-)₂-Y-(-CH₂-)- stehen, mit Y = Sauerstoff, Schwefel oder N-C₁-C₆-Alkyl und
- R^{1'} und R²: unabhängig von R¹ und R² den gleichen Bedeutungsumfang wie R¹ und R² aufweisen.

In dem erfindungsgemäßen Verfahren werden bevorzugt Diamide der Formel (I) eingesetzt, bei denen R¹ und R² einerseits und R^{1'} und R^{2'} andererseits gleich sind.

Weiterhin werden in das erfindungsgemäße Verfahren vorzugsweise Diamide eingesetzt, bei denen in Formel (II) R⁴, R⁵, R⁶ und R⁷ unabhängig voneinander jeweils für Wasserstoff oder Methyl stehen. Besonders bevorzugt stehen R⁴ und R⁶ für Wasserstoff und R⁵ und R⁷ für Wasserstoff oder Methyl. Ganz besonders bevorzugt sind R⁴, R⁵, R⁶ und R⁷ jeweils Wasserstoff.

Weiterhin werden in das erfindungsgemäße Verfahren vorzugsweise Diamide eingesetzt, bei denen in Formel (II) X für Sauerstoff, m für Null und n für Null oder 1 steht.

Weiterhin werden in das erfindungsgemäße Verfahren bevorzugt Diamide der Formel (I) eingesetzt, bei der R¹, R², R^{1'} und R^{2'} für Methyl oder Ethyl stehen oder R¹ und R² sowie R^{1'} und R^{2'} jeweils gemeinsam eine -(-CH₂-)₃-, -(-CH₂-)₄- oder -N(CH₃)-(-CH₂-)₂-Brücke bedeuten.

Besonders bevorzugt eingesetzt werden folgende Diamide 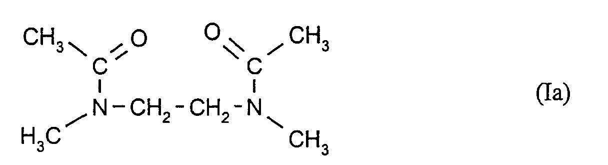 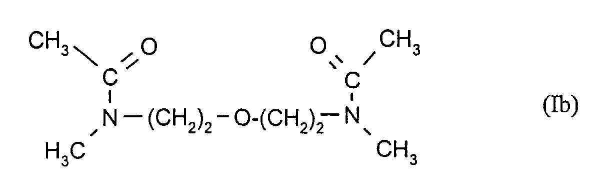 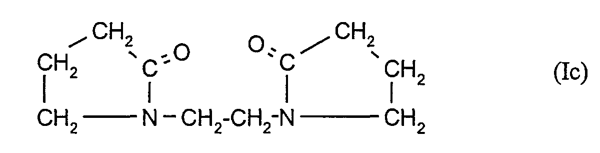 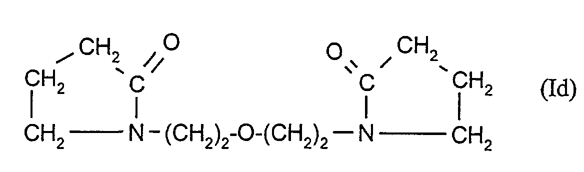 und

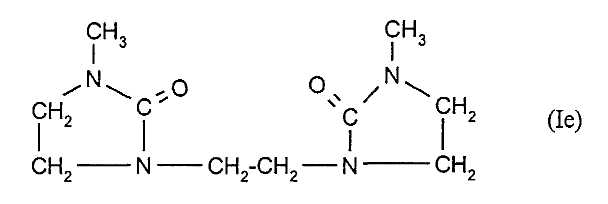

Die erfindungsgemäß einzusetzenden Diamide können als Einzelverbindung, als Gemische untereinander oder als Gemische mit bekannten Lösungsmitteln wie Dimethylformamid, Dimethylacetamid, N-Methylpyrrolidon, Tetramethylensulfon und/oder Dimethylsulfoxid eingesetzt werden. Vorzugsweise besteht beim erfindungsgemäßen Verfahren das eingesetzte Lösungsmittel zu mindestens 5 Mol-% aus Diamiden.

Im erfindungsgemäßen Verfahren kann man beispielsweise aromatische Chlor- oder Bromverbindungen der Formel (III) einsetzen 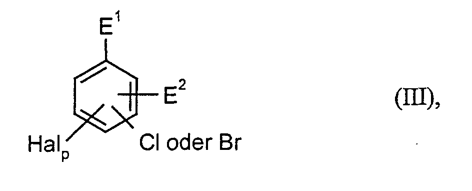 in der
- E¹: für einen elektronenziehenden Substituenten steht und
- E²: für Wasserstoff oder einen elektronenziehenden Substituenten steht oder
- E¹ und E²: ortho-ständig zueinander sind und gemeinsam für -CO-N-(C₁-C₆-Alkyl)-CO-stehen,
- Hal: für Fluor, Chlor oder Brom steht und
- P: Null oder eine ganze Zahl von 1 bis 3 bedeutet.

E¹ und E², E² soweit es für einen elektronenziehenden Substituenten steht, können unabhängig voneinander jeweils z.B. NO₂, CN, CF₃, CHO, COO-C₁-C₆-Alkyl, COZ oder SO₂Z (mit Z = Fluor, Chlor oder Brom) bedeuten.

E¹ und E² stehen vorzugsweise meta-ständig zueinander und vorzugsweise ortho- oder para-ständig zu Cl oder Br.

Wenn E¹ und/oder E² für einen COZ- oder SO₂Z-Rest steht, bei dem Z von Fluor verschieden ist, können bei der Durchführung des erfindungsgemäßen Verfahrens nicht nur Kern-Chloratome, sondern auch Z-Atome durch Fluor ersetzt werden. So kann man beispielsweise ausgehend von 2,4-Dichlorbenzoylchlorid auf die erfindungsgemäße Weise 2,4-Difluorbenzoylfluorid herstellen.

Liegen im Einsatzmaterial der Formel (III) mehrere Kernchlor- oder -bromatome vor, so können diese ganz oder teilweise gegen Fluor ausgetauscht werden. Dies ist abhängig von der Stellung der Chlor- bzw. Bromatome zu E¹ und E² und von der Menge des Fluorierungsmittels.

Im erfindungsgemäßen Verfahren setzt man pro auszutauschendes Chlor- oder Bromäquivalent setzt man vorzugsweise 0,9 bis 2 Mole Kaliumfluorid ein.

Das erfindungsgemäß einzusetzende Lösungsmittel kann beispielsweise in Mengen von 0,1 bis 5 Mol, bezogen auf 1 Mol der Verbindung der Formel (III) eingesetzt werden.

Geeignete Temperaturen für die Fluorierungsreaktion sind beispielsweise solche im Bereich von 150 bis 220°C. Gegebenenfalls kann man die Fluorierungsreaktion in einem geschlossenen Gefäß oder unter Druck durchführen, z.B. dann, wenn die Reaktionstemperatur in der Nähe oder über dem Siedepunkt der eingesetzten Verbindung der Formel (III) oder deren Fluorierungsprodukten liegt.

Man kann die Fluorierung gegebenenfalls in Gegenwart eines Katalysators durchführen. Als Katalysatoren kommen z.B. sogenannte Phasentransferkatalysatoren in Frage, bei denen es sich z.B. um Ammonium- oder Phosphoniumverbindungen handeln kann. Vorzugsweise arbeitet man ohne Katalysatorzusätze.

Das nach der erfindungsgemäßen Umsetzung vorliegende Reaktionsgemisch kann man z.B. so aufarbeiten, daß man zunächst die erhaltene, am Kern fluorierte aromatische Verbindung bei normalem oder erniedrigtem Druck abdestilliert. Aus dem hinterbleibenden Rückstand kann man das als Lösungsmittel eingesetzte Diamid gegebenenfalls durch Extraktion oder Destillation zurückgewinnen und erneut verwenden.

Die erfindungsgemäß einzusetzenden Diamide gestatten die Durchführung von Halex-Reaktionen zur Fluorierung von aromatischen Halogenverbindungen bei tieferen Temperaturen, auf einfachere Weise, ohne Risiko von spontanen Zersetzungen, bei kurzen Reaktionszeiten und mit guten Ausbeuten.

Die vorliegende Erfindung betrifft auch Diamide der Formel (I) 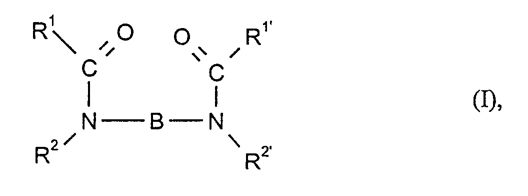 in der
- B: für eine Brücke der Formel (II) steht 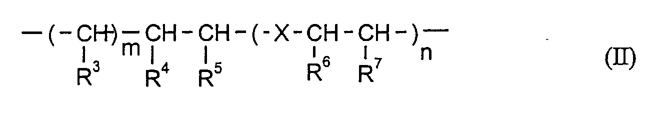 mit R³, R⁴, R⁵, R⁶ und R⁷ unabhängig voneinander jeweils gleich Wasserstoff oder C₁-C₆-Alkyl,
X= Sauerstoff, Schwefel oder N-C₁-C₆-Alkyl,
m= Null oder 1 und
n= Null, 1 oder 2,
- R¹ und R²: unabhängig voneinander für C₁-C₆-Alkyl oder gemeinsam für eine Brücke der Formeln
-(-CH₂-)₃-, -(-CH₂-)₄-, -Y-(-CH₂-)₂-, -Y-(-CH₂-)₃-, -(-CH₂-)-Y-(-CH₂-)-, -(-CH₂-)-Y-(-CH₂-)₂- oder -(-CH₂-)₂-Y-(-CH₂-)- stehen,
mit Y = Sauerstoff, Schwefel oder N-C₁-C₆-Alkyl und
- R^{1'} und R²': unabhängig von R¹ und R² den gleichen Bedeutungsumfang wie R¹ und R² aufweisen,
mit Ausnahme der Verbindungen, bei denen B für (CH₂)₂ steht
und R¹ Methyl, n-Propyl, iso.-Butyl, n-Pentyl, n-Hexyl oder 2-Ethyl-Butyl bedeutet
und R² Methyl bedeutet
und R¹ = R¹' und R² = R^{2'} ist
und Verbindungen, bei denen B für (CH₂)₃ steht
und R¹ = R¹' = R²= R²' Methyl ist
und Verbindungen, bei denen B für (CH₂)₂ steht
und R¹ Methyl ist
und R² iso-Propyl oder iso-Butyl bedeutet
und R¹ = R¹ und R² = R^{2'} ist
und Verbindungen, bei denen B für (CH-CH₃)₂ steht
und R¹ Methyl ist
und R² Ethyl bedeutet
und R¹ = R^{1'} und R² = R^{2'} ist
und Verbindungen, bei denen B für (CH₂)₂ oder (CH₂)₂-O-(CH₂)₂ steht
und R¹ + R² gemeinsam für eine Brücke der Formel (-CH₂-)₃ stehen
und R¹ + R² = R^{1'} + R^{2'} ist.

Bevorzugte Diamide sind solche der Formel (I), worin B eine Brücke der Formel (IV) bedeutet,

-CHR⁴-CHR⁵-(-X-CHR⁶-CHR⁷-)ₙ- (IV)

bei der
- R⁴, R⁵, R⁶ und R⁷: unabhängig voneinander für Wasserstoff oder Methyl,
- X: für Sauerstoff und
- n: für 0 oder 1 steht und

R¹, R², R¹' und R²' gleich sind und
für n = 0 und R⁴, R⁵ = Wasserstoff für Ethyl stehen und
für n = 0 oder 1 für Methyl oder Ethyl stehen oder
R¹ und R² sowie R¹' und R²' jeweils gemeinsam für eine -(CH₂-)₃-, -(CH₂-)₄- oder -N(CH₃)-(CH₂)₂-Brücke stehen, mit Ausnahme der Verbindungen, bei denen B für (CH₂)₂ oder (CH₂)₂-O-(CH₂)₂ steht und R¹ und R² sowie R¹' und R²' jeweils gemeinsam für eine -(CH₂-)₃-Brücke stehen.

Besonders bevorzugte erfindungsgemäße Diamide entsprechen den Formeln (Ib) und (Ie).

Erfindungsgemäße und in das erfindungsgemäße Fluorierungsverfahren einsetzbare Diamide kann man z.B. herstellen, indem man 1 Mol einer Verbindung der Formel Cl-B-Cl, in der B die bei Formel (II) angegebene Bedeutung hat, mit 2 Molen eines Amins der Formel R¹-CO-NH-R², in der R¹ und R² die bei Formel (I) angegebene Bedeutung haben, umsetzt. Dabei kann man unter Zusatz von Basen, in Gegenwart von Lösungsmitteln und unter Ausschleusung des entstehenden Wassers arbeiten.

Man kann auch 1 Mol einer Verbindung der Formel R²-NH-B-NH-R², in der R² für C₁-C₆-Alkyl steht und B die bei Formel (II) angegebene Bedeutung hat, mit 2 Molen einer Verbindung der Formel C₁-C₆-Alkyl-CO-Cl umsetzen. Dieses Verfahren eignet sich zur Herstellung von Diamiden der Formel (I), bei denen R¹, R², R^{1'} und R^{2'} für C₁-C₆-Alkyl steht.

Diamide der Formel (I), bei denen R¹ und R² sowie R^{1'} und R^{2'} jeweils eine Brücke der oben angegebenen Formeln bilden, kann man herstellen, indem man 1 Mol einer Verbindung der Formel H₂N-B-NH₂, in der B die bei Formel (II) angegebene Bedeutung hat, zunächst mit 2 Molen einer Verbindung der Formel Cl-Q-COCl, in der Q eine Brücke der Formel bedeutet, wie sie R¹ und R² gemeinsam bilden können, umsetzt, wobei eine Zwischenverbindung der Formel

Cl-Q-CO-NH-B-NH-CO-Q-Cl

entsteht und diese Zwischenverbindung mit starkem Alkali in eine Verbindung der Formel (I) überführen, bei der R¹ und R² sowie R^{1'} und R^{2'} eine Brücke der oben angegebenen Formeln bilden.

Weiterhin kann man auch 1 Mol einer Verbindung der Formel

C₁-C₆-Alkyl-NH-B-NH-C₁-C₆-Alkyl

mit einem Überschuß eines Anhydrids der Formel

C₁-C₆-Alkyl-CO-O-CO-C₁-C₆-Alkyl

umsetzen. So lassen sich Verbindungen der Formel (I) herstellen, bei denen R¹, R², R¹, und R²' für C₁-C₆-Alkyl stehen.

### Beispiele

### Beispiel 1 (nicht erfindungsgemäß)

### Herstellung der Verbindung der Formel (Ia)

106,5 ml 1,2-Bis-(methylamino)-ethan wurden unter Eiskühlung vorgelegt und dazu im Verlauf von 2 Stunden 377,5 ml Essigsäureanhydrid zugetropft. Anschließend wurde 4 Stunden bei Raumtemperatur nachgerührt. Das erhaltene Reaktionsgemisch wurde durch fraktionierte Destillation im Vakuum aufgearbeitet. Bei 125 bis 130°C/0,75 mbar wurde die Verbindung der Formel (Ia) abgetrennt und anschließend aus Benzin (Siedebereich 80 bis 110°C) umkristallisiert. Es wurden 193,0 g (= 89,3 % d. Th.) Produkt mit einem Schmelzpunkt von 92,5°C erhalten.

### Beispiel 2 (nicht erfindungsgemäß)

### Herstellung der Verbindung der Formel (Id)

106,5 ml 2-Pyrrolidinon wurden in 730ml Dimethylsulfoxid vorgelegt und 94,1 g gepulvertes Kaliumhydroxid und 3,0g N-Benzyl-4-N,N'-dimethylaminopyridiniumchlorid (Katalysator) zugefügt. Unter Eiskühlung wurden im Verlauf von 45 Minuten 82,0 ml Bis-(2-chlorethyl)-ether zugetropft und 60 Stunden bei Raumtemperatur nachgerührt. Aus dem erhaltenen Reaktionsgemisch wurde zunächst das gebildete Kaliumchlorid abfiltriert und mit 300 ml Tetrahydrofuran gewaschen. Das Filtrat und die Waschflüssigkeit wurden vereinigt, die Lösungsmittel im Vakuum abgetrennt, erneut ausfallendes Kaliumchlorid abfiltriert und das Rohprodukt fraktioniert destilliert. Bei 168 bis 170°C/0,5 mbar wurden 143,0 g (= 85,47 % d.Th.) der Verbindung der Formel (Id) in Form eines gelben Öls erhalten. Das Produkt wies einen Brechungsindex n = 1,5089 auf, und das ¹H-NMR-Spektrum (200 MHz DMSO) wies charakteristische Banden bei δ = 1,95, 2,24, 3,36, 3,41 und 3,53 ppm auf, das IR-Spektrum bei 2900, 1680, 1493, 1460, 1300 und 1134 cm⁻¹.

### Beispiel 3

### Herstellung der Verbindung der Formel (Ib)

110,0 g N-Methylacetamid, 101,0 g Kaliumhydroxid und 750 ml Dimethylsulfoxid wurden bei Raumtemperatur gemischt und 45 Minuten gerührt. Nach Zugabe von 3,0 g N-Benzyl-4-N,N'-dimethylaminopyridiniumchlorid (Katalysator) und Kühlung des Gemisches auf 10°C wurden im Verlauf von 55 Minuten 88,0 ml Bis-(2-chlorethyl)-ether zugetropft und danach 40 Stunden bei Raumtemperatur nachgerührt. Die Aufarbeitung des Reaktionsgemisches erfolgte wie in Beispiel 2 beschrieben. Bei der Destillation fielen drei die Verbindung der Formel (Ib) enthaltende Fraktionen an: a) 133 bis 138°C/0,5 mbar, 81,9 %ig - 21,0 g, b) 138 bis 142°C/0,5 mbar, 100 %ig - 11,0 g und c) 142 bis 144°C/0,5 mbar, 98,0 %ig - 4,0 g.

An der Fraktion b) wurden folgende Parameter gemessen: Brechungsindex n = 1,4842, ¹H-NMR-Spektrum (200 MHz, CDCl₃) mit charakteristischen Banden bei δ = 2,09, 2,11, 2,12, 2,94, 3,04, 3,07 und 3,54 ppm und IR-Spektrum mit charakteristischen Banden bei 2900, 1480, 1440 bis 1410, 1360, 1125 und 1030 cm⁻¹.

### Beispiel 4 (nicht erfindungsgemäß)

### Herstellung der Verbindung der Formel (Ic)

a) 1. Stufe
   Eine Mischung aus 20,0 ml 1,2-Diaminoethan und 200 ml Toluol wurde unter Eiskühlung vorgelegt und dazu im Verlauf von 2 Stunden 56,0 ml 4-Chlorbuttersäurechlorid, gelöst in 400 ml Toluol, zugetropft. Der freiwerdende Chlorwasserstoff wurde durch Zusatz von 23,75 g Pyridin gebunden. Nach 2-stündigem Rühren bei Raumtemperatur wurde das Reaktionsgemisch abgesaugt, mit 600 ml Wasser gewaschen und der Filterkuchen bei 70°C getrocknet. So wurden 75,0 g (= 93,0 % d.Th.) N,N'-Di-4-chlor-1-oxobutyl-1,2-diaminoethan in Form eines weißen Feststoffs mit dem Schmelzpunkt 138 bis 139,5°C erhalten.
b) 2. Stufe
   135,3 g Kaliumhydroxid wurden in 4,0 1 Toluol auf 60°C erhitzt. Nach der Zugabe von 3,0 g N-Benzyl-4-N,N'-dimethylaminopyridiniumchlorid (Katalysator) und 243,0 g des in der 1. Stufe erhaltenen Produkts stieg die Innentemperatur auf 98 bis 100°C an, wobei der in der 1. Stufe erhaltene Feststoff unter Gelbfärbung in Lösung ging. Bei dieser Temperatur wurde 1 Stunde am Rückfluß nachgerührt. Dann wurde abgekühlt und feste Bestandteile (im wesentlichen Kaliumchlorid) abfiltriert. Aus dem Filtrat wurde das Lösungsmittel im Vakuum abgezogen, wobei ein Rohprodukt mit einer Reinheit von 97,2 % in Form hellgelber Nadeln ausfiel. Diese wurden abfiltriert und aus Cyclohexan umkristallisiert. So wurden 84,0 g (47,2 % d.Th.) der Verbindung der Formel (Ic) in Form farbloser Nadeln mit einem Schmelzpunkt von 106 bis 108°C erhalten. Das ¹H-NMR-Spektrum (200 MHz, CDCl₃) zeigte charakteristische Banden bei δ = 1,99, 2,32, 3,45 und 3,48 ppm, daß IR-Spektrum bei 2930, 1670, 1467, 1430 und 1298 cm⁻¹.

### Beispiel 5

### Fluorierung mit der Verbindung der Formel (Ia)

113,0 g der Verbindung der Formel (Ia) wurden mit 36,3 g Kaliumfluorid versetzt und aus dieser Mischung Spuren von Wasser durch Andestillieren entfernt. Dann wurden 96,0 g 3,4-Dichlornitrobenzol zugefügt und 5,75 Stunden bei 190°C gerührt. Aus dem Reaktionsgemisch wurden dann durch Wasserdampfdestillation 82,0 g einer organischen Phase erhalten, die zu 97,5 % aus 3-Chlor-4-fluor-nitrobenzol bestand. Das entspricht einer Ausbeute von 91,2 % d.Th.

### Vergleich

Beispiel 5 wurde wiederholt, jedoch wurde als Lösungsmittel eine entsprechende Menge Tetramethylensulfon eingesetzt. Nach einer Reaktionszeit von 22 Stunden wurde 3-Chlor-4-fluor-nitrobenzol in einer Ausbeute von 32,8 % d.Th. erhalten.

### Beispiel 6

### Fluorierung mit der Verbindung der Formel (Ic)

97,0 g der Verbindung der Formel (Ic) wurden mit 27,4 g Kaliumfluorid versetzt und aus dieser Mischung Spuren von Wasser durch Andestillieren entfernt. Dann wurden 72,5 g 3,4-Dichlornitrobenzol zugefügt und 6 Stunden bei 190°C gerührt. Aus dem Reaktionsgemisch wurden dort vorhandene Salze durch Filtration abgetrennt und mit 400 ml Methylenchlorid gewaschen. Das Filtrat und die Waschflüssigkeit wurden vereinigt, im Vakuum eingeengt und nach Zugabe von 300 ml Wasser einer Wasserdampfdestillation unterworfen. Es wurden 46,4 g 3-Chlor-4-fluor-nitrobenzol erhalten. Das entspricht einer Ausbeute von 70,0 % d.Th.

### Beispiel 7

### Fluorierung mit der Verbindung der Formel (Id)

113,0 g der Verbindung der Formel (Id) wurden mit 25,2 g Kaliumfluorid versetzt und aus dieser Mischung Spuren von Wasser durch Andestillieren entfernt. Dann wurden 69,2 g 3,4-Dichlornitrobenzol zugefügt und 4,5 Stunden bei 190°C gerührt. Die Aufarbeitung des Reaktionsgemisches erfolgte wie in Beispiel 6 angegeben. Es wurden 30,3 g 3-Chlor-4-fluor-nitrobenzol erhalten. Das entspricht einer Ausbeute von 48,1 % d.Th.

### Beispiel 8

Fluorierung mit einem Gemisch der Verbindung der Formel (Ib) mit Tetramethylensulfon

20,0 g der Verbindung der Formel (Ib) wurden mit 24,0 g Sulfolan und 18,6 g Kaliumfluorid versetzt und aus dieser Mischung Spuren von Wasser durch Andestillieren entfernt. Dann wurden 51,9 g 3,4-Dichlornitrobenzol zugesetzt und der Ansatz 12,75 Stunden bei 190°C gerührt. Die Aufarbeitung des Reaktionsgemischs erfolgte wie in Beispiel 5 angegeben. Es wurden 29,84 g 3-Chlor-4-fluor-nitrobenzol erhalten. Das entspricht einer Ausbeute von 63,5 % d.Th.

### Beispiel 9

Fluorierung mit einem Gemisch der Verbindung der Formel (Ia) mit Tetramethylensulfon

278 g Kaliumfluorid wurden in 600 ml Tetramethylensulfon und 42 g der Verbindung (Ia) suspendiert und bei 15 mbar andestilliert. Anschließend wurden 299 g N-Methyltetrachlorphthalimid zugegeben und unter Feuchtigkeitsauschluß für 5 Stunden bei 165°C gerührt. Danach wurde mittels GC-Analyse festgestellt, daß mehr als 98 % des Ausgangsmaterials zum N-Methyl-tetrafluorphthalsäureimid fluoriert worden waren. Das Reaktionsgemisch wurde in 2 l Wasser eingerührt, der ausgefallene Niederschlag abgesaugt, mit Wasser nachgewaschen und getrocknet. Die Ausbeute betrug 215 g = 92 % d.Th. (Schmelzpunkt: 132°C).

### Beispiel 10 (zum Vergleich)

Es wurde verfahren wie in Beispiel 9, jedoch wurde keine Verbindung der Formel (Ia) verwendet. Die GC-Analyse nach 5 Stunden bei 165°C zeigte an, daß insgesamt 18 Gew.-% nicht vollständige fluorierte Verbindungen im Reaktionsgemisch vorlagen. Nach weiterem 3-stündigem Rühren bei 190°C war alles Ausgangsmaterial und alle noch nicht vollständig fluorierten Verbindungen zu N-Methyl-tetrafluorphthalsäureimid abreagiert.

### Beispiel 11

### Fluorierung mit einem Gemisch der Verbindung der Formel (Ia) mit Tetramethylensulfon

Zu einer Suspension aus 240 g Kaliumfluorid in 380 ml Tetramethylensulfon wurden 60 g der Verbindung (Ia) gegeben und 50 ml Xylol zugesetzt. Aus dieser Mischung wurden Spuren von Wasser durch Abdestillieren zusammen mit dem Xylol entfernt. Anschließend wurden 284 g 2-H-Tetrachlorbenzotrifluorid zugegeben und das Gemisch unter Feuchtigkeitsausschluß für 8 Stunden bei 190°C gerührt. Dann wurden die flüchtigen Bestandteile des Reaktionsgemisches unter reduziertem Druck abdestilliert und das Destillat einer Feindestillation bei Normaldruck unterworfen. Es wurden 108 g 5-Chlor-2,3,4-trifluorbenzotrifluorid mit einem Siedepunkt von 134-135°C und einem Brechungsindex von n = 1,4120 erhalten.

### Beispiel 12 (zum Vergleich)

Es wurde verfahren wie in Beispiel 11, jedoch wurde keine Verbindung der Formel (Ia) verwendet. Die Ausbeute an 5-Chlor-2,3,4-trifluorbenzotrifluorid betrug 67 g.

## Patentansprüche

1. Verfahren zur Herstellung von aromatischen, am Kern fluorierten Verbindungen durch Umsetzung von entsprechenden Chlor- oder Bromverbindungen mit Kaliumfluorid in einem Lösungsmittel, **dadurch gekennzeichnet, daß** man als Lösungsmittel ein Diamid einsetzt.

2. Verfahren nach Anspruch 1, **dadurch gekennzeichnet, daß** man ein Diamid der Formel (I) einsetzt 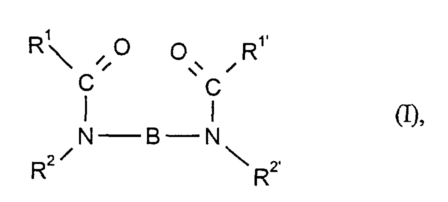 in der
B für eine Brücke der Formel (II) steht 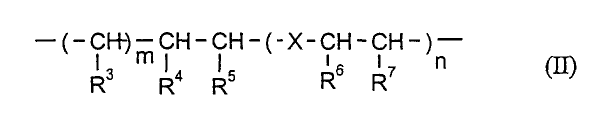 mit R³, R⁴, R⁵, R⁶ und R⁷ unabhängig voneinander jeweils gleich
Wasserstoff oder C₁-C₆-Alkyl,
X= Sauerstoff, Schwefel oder N-C₁-C₆-Alkyl,
m= Null oder 1 und
n= Null, 1 oder 2,
R¹ und R² unabhängig voneinander für C₁-C₆-Alkyl oder gemeinsam für eine Brücke der Formeln
-(-CH₂-)₃-, -(-CH₂-)₄-, -Y-(-CH₂-)₂-, -Y-(-CH₂-)₃-, -(-CH₂-)-Y-(-CH₂-)-, -(-CH₂-)-Y-(-CH₂-)₂- oder -(-CH₂-)₂-Y-(-CH₂-)- stehen,
mit Y= Sauerstoff, Schwefel oder N-C₁-C₆-Alkyl und
R^{1'} und R^{2'} unabhängig von R¹ und R² den gleichen Bedeutungsumfang wie R¹ und R² aufweisen.

3. Verfahren nach Anspruch 2, **dadurch gekennzeichnet, daß** man Diamide einsetzt, bei denen in Formel (II) R⁴, R⁵, R⁶ und R⁷ unabhängig voneinander jeweils für Wasserstoff oder Methyl, X für Sauerstoff, m für Null und n für Null oder 1 stehen und in Formel (I) R¹, R², R^{1'} und R^{2'} für Methyl oder Ethyl stehen oder R¹ und R² sowie R^{1'} oder R^{2'} jeweils gemeinsam eine
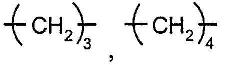 oder - N(CH₃) 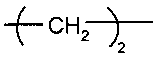 Brücke bedeuten.

4. Verfahren nach Ansprüchen 1 bis 3, **dadurch gekennzeichnet, daß** man ein Diamid der Formeln (Ia) bis (Ie) 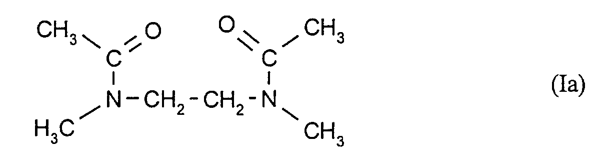 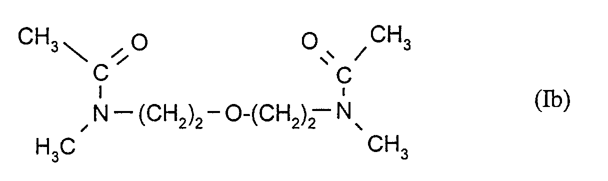 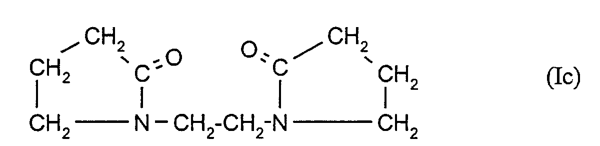 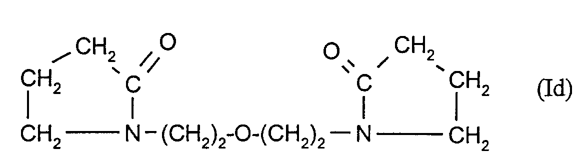 oder 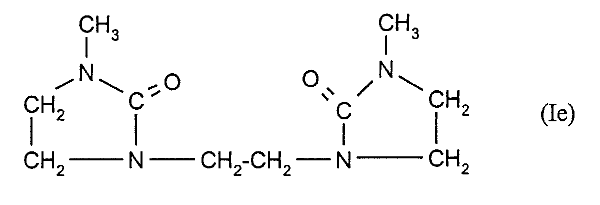 einsetzt.

5. Verfahren nach Ansprüchen 1 bis 4, **dadurch gekennzeichnet, daß** man aromatische Chlor- oder Bromverbindungen der Formel (III) einsetzt 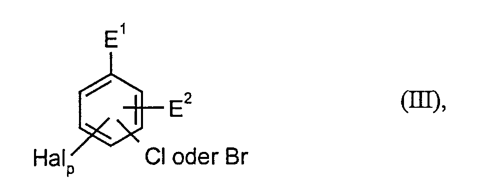 in der
E¹ für einen elektronenziehenden Substituenten steht und
E² für Wasserstoff oder einen elektronenziehenden Substituenten steht oder
E¹ und E² ortho-ständig zueinander sind und gemeinsam für -CO-N-(C₁-C₆-Alkyl)-CO- stehen,
Hal für Fluor, Chlor oder Brom steht und
p Null oder eine ganze Zahl von 1 bis 3 bedeutet.

6. Verfahren nach Anspruch 5, **dadurch gekennzeichnet, daß** in Formel (III) E¹ und E², E² soweit es für einen elektronenziehenden Substituenten steht, unabhängig voneinander jeweils NO₂, CN, CF₃, CHO, COO-C₁-C₆-Alkyl, COZ oder SO₂Z (mit Z = Fluor, Chlor oder Brom) bedeutet.

7. Verfahren nach Ansprüchen 1 bis 6, **dadurch gekennzeichnet, daß** man pro auszutauschendes Chlor- oder Brom-Äquivalent 0,9 bis 2 Mole Kaliumfluorid einsetzt.

8. Verfahren nach Ansprüchen 1 bis 7, **dadurch gekennzeichnet, daß** man es bis 150 bis 220°C durchführt.

9. Diamide der Formel (I) 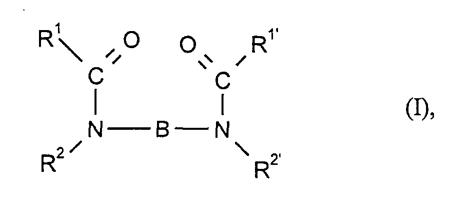 in der
B für eine Brücke der Formel (II) steht 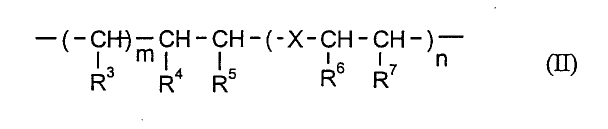 mit R³, R⁴, R⁵, R⁶ und R⁷ unabhängig voneinander jeweils gleich Wasserstoff oder C₁-C₆-Alkyl,
X= Sauerstoff, Schwefel oder N-C₁-C₆-Alkyl,
m = Null oder 1 und
n = Null, 1 oder 2,
R¹ und R² unabhängig voneinander für C₁-C₆-Alkyl oder gemeinsam für eine Brücke der Formeln
-(-CH₂-)₃-, -(-CH₂-)₄-, -Y-(-CH₂-)₂-, -Y-(-CH₂-)₃-, -(-CH₂-)-Y-(-CH₂-)-, -(CH₂-)-Y-(-CH₂-)₂- oder -(-CH₂-)₂-Y-(-CH₂-)-stehen,
mit Y = Sauerstoff, Schwefel oder N-C₁-C₆-Alkyl und
R^{1'} und R^{2'}unabhängig von R¹ und R² den gleichen Bedeutungsumfang wie R¹ und R² aufweisen,
mit Ausnahme der Verbindungen, bei denen B für (CH₂)₂ steht
und R¹ Methyl, n-Propyl, iso.-Butyl, n-Pentyl, n-Hexyl oder 2-Ethyl-Butyl bedeutet
und R² Methyl bedeutet
und R¹ = R^{1'} und R² = R^{2'} ist
und Verbindungen, bei denen B für (CH₂)₃ steht
und R¹ = R^{1'} = R²= R^{2'} Methyl ist
und Verbindungen, bei denen B für (CH₂)₂ steht
und R¹ Methyl ist
und R² iso-Propyl oder iso-Butyl bedeutet
und R¹ = R¹ und R² = R^{2'} ist
und Verbindungen, bei denen B für (CH-CH₃)₂ steht
und R¹ Methyl ist
und R² Ethyl bedeutet
und R¹ = R¹' und R² = R²' ist
und Verbindungen, bei denen B für (CH₂)₂ oder (CH₂)₂-O-(CH₂)₂ steht
und R¹ + R² gemeinsam für eine Brücke der Formel (-CH₂-)₃ stehen
und R¹ + R2 = R¹' + R²' ist.

10. Diamide nach Anspruch 9, **dadurch gekennzeichnet, dass** in Formel (I) B eine Brücke der Formel (IV) bedeutet,
-CHR⁴-CHR⁵-(-X-CHR⁶-CHR⁷-)ₙ- (IV)
bei der
R⁴, R⁵, R⁶ und R⁷ unabhängig voneinander für Wasserstoff oder Methyl,
X für Sauerstoff und
n für 0 oder 1 steht und
R¹, R², R^{1'} und R^{2'} gleich sind und
für n = 0 und R⁴, R⁵ = Wasserstoff für Ethyl stehen und
für n = 0 oder 1 für Methyl oder Ethyl stehen oder
R¹ und R² sowie R¹' und R²' jeweils gemeinsam für eine -(CH₂-)₃-, -(CH₂-)₄-oder -N(CH₃)-(CH₂)₂-Brücke stehen, mit Ausnahme der Verbindungen, bei denen B für (CH₂)₂ oder (CH₂)₂-O-(CH₂)₂ steht und R¹ und R² sowie R¹' und R²' jeweils gemeinsam für eine -(CH₂-)₃-Brücke stehen.

## Claims

1. Process for the preparation of aromatic, ring-fluorinated compounds by reaction of corresponding chlorine compounds or bromine compounds with potassium fluoride in a solvent, **characterized in that** the solvent used is a diamide.

2. Process according to Claim 1, **characterized in that** a diamide of the formula (I) is used 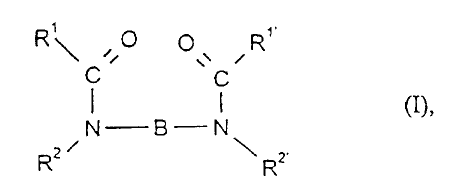 in which
B represents a bridge of the formula (II) 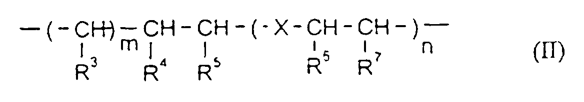 where R³, R⁴, R⁵, R⁶ and R⁷, independently of each other, are each hydrogen or C₁-C₆-alkyl,
X= oxygen, sulphur or N-C₁-C₆-alkyl,
m= zero or 1 and
n = zero, 1 or 2,
R¹ and R², independently of each other, represent C₁-C₆-alkyl or together represent a bridge of the formulae
-(-CH₂-)₃-, -(-CH₂-)₄-, -Y-(-CH₂-)₂-, -Y-(-CH₂-)₃-, -(-CH₂-)-Y-(-CH₂-)-, -(-CH₂-)-Y-(-CH₂-)₂- or -(-CH₂-)₂-Y-(-CH₂-)-,
where Y = oxygen, sulphur or N-C₁-C₆-alkyl and
R^{1'} and R^{2'}, independently of R¹ and R², have the same scope of meaning as R¹ and R².

3. Process according to Claim 2, **characterized in that** diamides are used in which in formula (II) R⁴, R⁵, R⁶ and R⁷, independently of each other, each represent hydrogen or methyl, X represents oxygen, m represents zero and n represents zero or 1 and in formula (I) R¹, R², R^{1'} and R^{2'} represent methyl or ethyl or R¹ and R², and R^{1'} or R^{2'}, each together denote a
-(-CH₂-)₃-, -(-CH₂-)₄- or -N(CH₃)-(-CH₂-)₂- bridge.

4. Process according to Claims 1 to 3, **characterized in that** a diamide is used of the formulae (Ia) to (Ie) 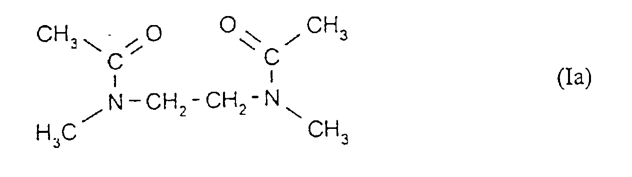 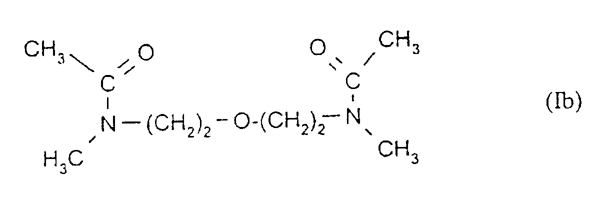 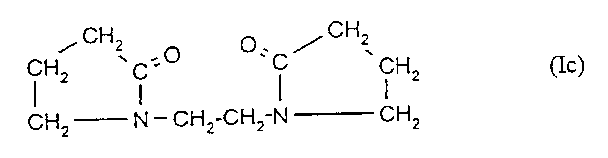 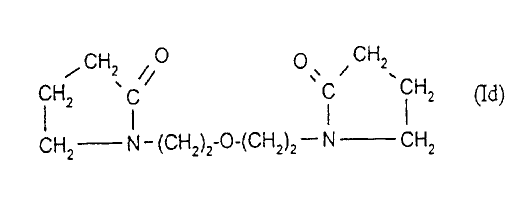 or

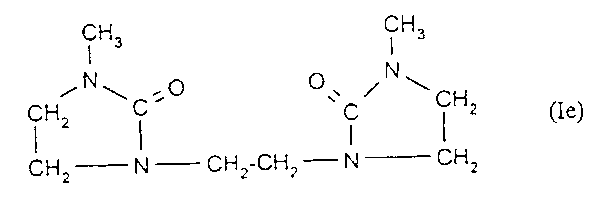

5. Process according to Claims 1 to 4, **characterized in that** aromatic chlorine compounds or bromine compounds of the formula (III) are used 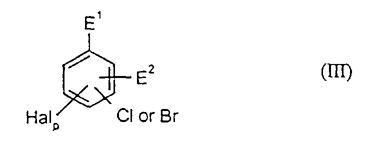 in which
E¹ represents an electron-withdrawing substituent and
E² represents hydrogen or an electron-withdrawing substituent or
E¹ and E² are in the ortho position to each other and together represent -CO-N-(C₁-C₆-alkyl)-CO-,
Hal represents fluorine, chlorine or bromine and
p denotes zero or an integer from 1 to 3.

6. Process according to Claim 5, **characterized in that** in formula (III) E¹ and E², E² if it represents an electron-withdrawing substituent, independently of each other, each denote NO₂, CN, CF₃, CHO, COO-C₁-C₆-alkyl, COZ or SO₂Z (where Z = fluorine, chlorine or bromine).

7. Process according to Claims 1 to 6, **characterized in that** per equivalent of chlorine or bromine to be exchanged, 0.9 to 2 mol of potassium fluoride are used.

8. Process according to Claims 1 to 7, **characterized in that** it is carried out at 150 to 220°C.

9. Diamides of the formula (I) 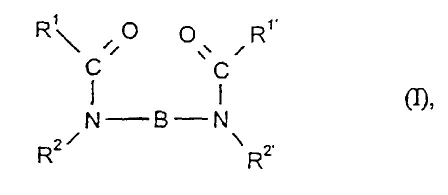 in which
B represents a bridge of the formula (II) 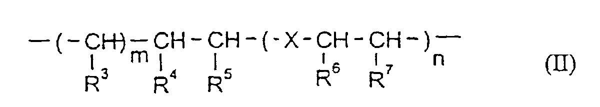 where R³, R⁴, R⁵, R⁶ and R⁷, independently of each other, are each hydrogen or C₁-C₆-alkyl,
X = oxygen, sulphur or N-C₁-C₆-alkyl,
m = zero or 1 and
n = zero, 1 or 2,
R¹ and R², independently of each other, represent C₁-C₆-alkyl or together represent a bridge of the formulae
-(-CH₂-)₃-, -(-CH₂-)₄-, -Y-(-CH₂-)₂-, -Y-(-CH₂-)₃-, -(-CH₂-)-Y-(-CH₂-)-, -(-CH₂-)-Y-(-CH₂-)₂- or -(-CH₂-)₂-Y-(-CH₂-)-,
where Y = oxygen, sulphur or N-C₁-C₆-alkyl and
R^{1'} and R^{2'}, independently of R¹ and R², have the same scope of meaning as R' and except the compounds in which B represents (CH₂)₂
and R¹ denotes methyl, n-propyl, iso-butyl, n-pentyl, n-hexyl or 2-ethyl-butyl
and R² denotes methyl
and R¹ = R^{1'} and R² = R^{2'}
and compounds in which B represents (CH₂)₃
and R¹ = R^{1'} = R² = R²' and is methyl
and compounds in which B represents (CH₂)₂
and R¹ is methyl
and R² denotes iso-propyl or iso-butyl
and R¹ = R^{1'} = R² = R^{2'}
and compounds in which B represents (CH-CH₃)₂
and R¹ is methyl
and R² denotes ethyl
and R¹ = R^{1'} and R² = R^{2'}
and compounds in which R represents (CH₂)₂ or (CH₂)₂-O-(CH₂)₂
and R¹ + R² together represent a bridge of the formula (-CH₂-)₃
and R¹ + R² = R^{1'} + R^{2'}.

10. Diamides according to Claim 9, **characterized in that** in formula (I) B represents a bridge of the formula (IV)
-CHR⁴-CHR⁵-(-X-CHR⁶-CHR⁷-)-ₙ (IV)
in which
R⁴, R⁵, R⁶ and R⁷, independently of each other, represent hydrogen or methyl,
X represents oxygen, and
n represents 0 or 1 and
R¹, R², R^{1'} and R^{2'} are identical and
represent ethyl where n = 0 and R⁴, R⁵ = hydrogen and
represent methyl or ethyl where n = 0 or 1 or
R¹ and R², and R' and R^{2'}, each together represent a -(-CH₂-)₃-, -(-CH₂-)₄- or -N(CH₃)-(CH₂)₂- bridge, except the compounds in which B represents (CH₂)₂ or (CH₂)₂-O-(CH₂)₂ and R¹ and R², and R^{1'} and R^{2'}, each together represent a -(CH₂-)₃ bridge.

## Revendications

1. Procédé de préparation de composés aromatiques fluorés sur le cycle par réaction de composés chlorés ou bromés correspondants avec du fluorure de potassium dans un solvant, **caractérisé en ce que** l'on utilise un diamide comme solvant.

2. Procédé selon la revendication 1, **caractérisé en ce que** l'on utilise un diamide de formule (I) 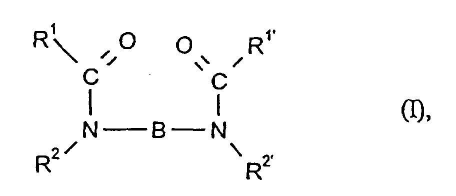 dans laquelle
B représente un pont de formule (II) 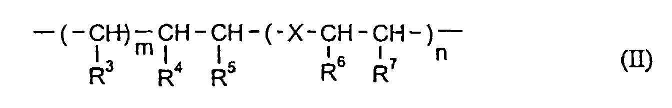 dans laquelle
R³, R⁴, R⁵, R⁶ et R⁷ représentent chacun indépendamment les uns des autres un atome d'hydrogène ou un groupe alkyle en C₁-C₆,
X est un atome d'oxygène ou de soufre ou un groupe N-alkyle en C₁-C₆,
m = 0 ou 1 et
n = 0,1 ou 2,
R¹ et R² représentent indépendamment l'un de l'autre un groupe alkyle en C₁-C₆ ou forment ensemble un pont de formule -(-CH₂-)₃-, -(-CH₂-)₄-, -Y-(-CH₂-)₂-, -Y-(-CH₂-)₃-, -(-CH₂-)-Y-(-CH₂-)-, -(-CH₂-)-Y-(-CH₂-)₂- ou -(-CH₂-)₂-Y-(-CH₂-)-,
où Y est un atome d'oxygène ou de soufre ou un groupe N-alkyle en C₁-C₆, et
R^{1'} et R^{2'}, indépendamment de R¹ et R², ont la même définition que R¹ et R².

3. Procédé selon la revendication 2, **caractérisé en ce que** l'on utilise des diamides dans lesquels, dans la formule (II), R⁴, R⁵, R⁶ et R⁷ représentent chacun, indépendamment les uns des autres, un atome d'hydrogène ou un groupe méthyle, X est un atome d'oxygène, m est 0 et n est 0 ou 1 et, dans la formule (I), R¹, R², R^{1'} et R²' représentent des groupes méthyle ou éthyle, ou R¹ et R², ainsi que R^{1'} et R²' forment ensemble des ponts -(-CH₂-)₃-, -(-CH₂-)₄- ou -N(CH₃)-(-CH₂-)₂-.

4. Procédé selon les revendications 1 à 3, **caractérisé en ce que** l'on utilise un diamide ayant l'une des formules (Ia) à (Ie) 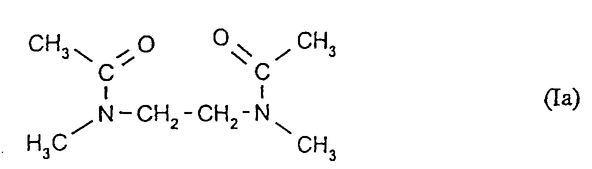 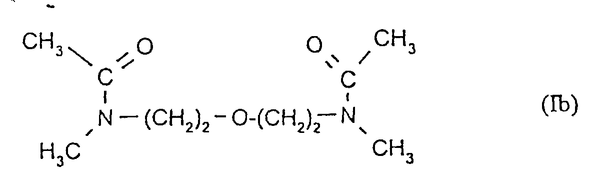 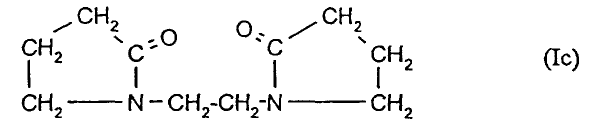 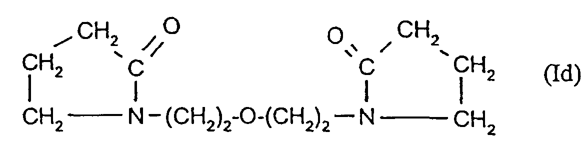 ou

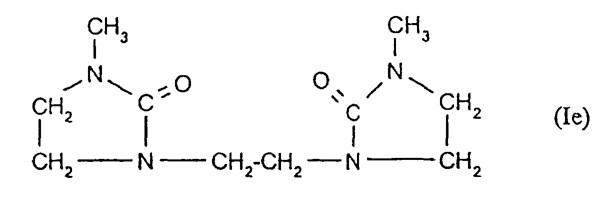

5. Procédé selon les revendications 1 à 4, **caractérisé en ce que** l'on utilise des composés aromatiques chlorés ou bromés de formule (III) 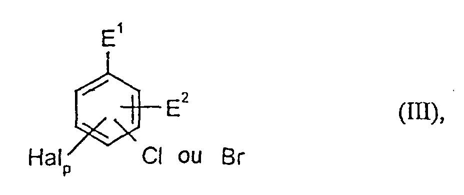 dans laquelle
E¹ représente un substituant attracteur d'électrons et
E² représente un atome d'hydrogène ou un substituant attracteur d'électrons, ou
E¹ et E² sont en position ortho l'un par rapport à l'autre et forment ensemble le groupe -CO-N-(alkyle en C₁-C₆)-CO-,
Hal représente le fluor, le chlore ou le brome et
p est 0 ou un nombre entier de 1 à 3.

6. Procédé selon la revendication 5, **caractérisé en ce que**, dans la formule (III), E¹ et E², E² dans la mesure où il représente un substituant attracteur d'électrons, peuvent représenter chacun indépendamment l'un de l'autre un groupe NO₂, CN, CF₃, CHO, COO-(alkyle en C₁-C₆), COZ ou SO₂Z (avec Z = fluor, chlore ou brome).

7. Procédé selon les revendications 1 à 6, **caractérisé en ce que** l'on utilise 0,9 à 2 mol de fluorure de potassium par équivalent de chlore ou de brome à échanger.

8. Procédé selon les revendications 1 à 7, **caractérisé en ce qu'**on le met en oeuvre à une température de 150 à 220°C.

9. Diamides de formule (I) 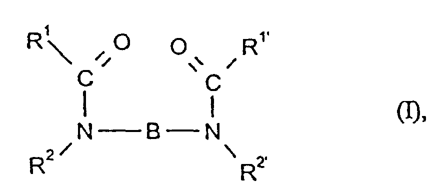 dans laquelle
B représente un pont de formule (II) 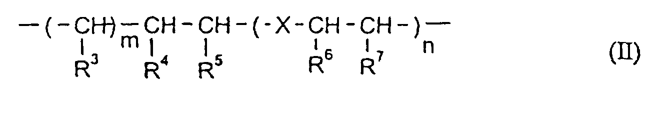 dans laquelle
R³, R⁴, R⁵, R⁶ et R⁷ représentent chacun indépendamment les uns des autres un atome d'hydrogène ou un groupe alkyle en C₁-C₆,
X est un atome d'oxygène ou de soufre ou un groupe N-alkyle en C₁-C₆,
m = 0 ou 1 et
n = 0,1 ou 2,
R¹ et R² représentent indépendamment l'un de l'autre un groupe alkyle en C₁-C₆ ou forment ensemble un pont de formule -(-CH₂-)₃-, -(-CH₂-)₄-, -Y-(-CH₂-)₂-, -Y-(-CH₂-)₃-, -(-CH₂-)-Y-(-CH₂-)-, -(-CH₂-)-Y0-(-CH₂-)₂- ou -(-CH₂-)₂-Y-(-CH₂-)-,
où Y est un atome d'oxygène ou de soufre ou un groupe N-alkyle en C₁-C₆, et R^{1'} et R²', indépendamment de R¹ et R², ont la même définition que R¹ et R², à l'exception des composés dans lesquels B représente (CH₂)₂
et R¹ est un groupe méthyle, n-propyle, isobutyle, n-pentyle, n-hexyle ou 2-éthylbutyle,
et R² représente un groupe méthyle,
et R¹= R^{1'} et R² = R^{2'},
et des composés dans lesquels B représente (CH₂)₃
et R¹ = R² = R^{1'} = R^{2'} = méthyle,
et des composés dans lesquels B représente (CH₂)₂,
et R¹ est un groupe méthyle
et R² est un groupe isopropyle ou isobutyle
et R¹ = R^{1'} et R² = R^{2'},
et des composés dans lesquels B représente (CH-CH₃)₂
et R¹ est un groupe méthyle
et R² est un groupe éthyle
et R¹ = R^{1'} et R² = R²',
et des composés dans lesquels B représente (CH₂)₂ ou (CH₂)₂-O-(CH₂)₂
et R¹ + R² forment ensemble un pont de formule (-CH₂-)₃
et R¹ + R² = R^{1'} + R²'.

10. Diamides selon la revendication 9, **caractérisés en ce que**, dans la formule (I), B représente un pont de formule (IV)
-CHR⁴-CHR⁵-(-X-CHR⁶-CHR⁷-)ₙ- (IV)
dans laquelle
R⁴, R⁵, R⁶ et R⁷ représentent chacun indépendamment les uns des autres un atome d'hydrogène ou un groupe méthyle,
X représente l'oxygène,
n est 0 ou 1, et
R¹, R², R^{1'} et R²' sont identiques et représentent un groupe éthyle lorsque n = 0 et R⁴ et R⁵ sont des atomes d'hydrogène, et un groupe méthyle ou éthyle lorsque n = 0 ou 1, ou
R¹ et R², ainsi que R^{1'} et R^{2'} forment ensemble des ponts -(-CH₂-)₃-, -(-CH₂-)₄- ou -N(CH₃)-(-CH₂-)₂-, à l'exception des composés dans lesquels B est un groupe (CH₂)₂ ou (CH₂)₂-O-(CH₂)₂, et R¹ et R², ainsi que R^{1'} et R²' forment ensemble des ponts -(-CH₂-)₃-.
